(19) Europäisches Patentamt
European Patent Office
Office européen des brevets

(11) **EP 3 884 966 A2**

(12) **DEMANDE DE BREVET EUROPEEN**

(43) Date de publication:
**29.09.2021 Bulletin 2021/39**

(51) Int Cl.:
*A61L 2/10* (2006.01)    *A23L 3/00* (2006.01)
*A61K 9/00* (2006.01)    *B65B 55/00* (2006.01)
*H02K 33/00* (2006.01)

(21) Numéro de dépôt: **21165194.8**

(22) Date de dépôt: **26.03.2021**

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**
Etats d'extension désignés:
**BA ME**
Etats de validation désignés:
**KH MA MD TN**

(30) Priorité: **26.03.2020 FR 2002963**

(71) Demandeur: **Sterixene
30133 Les Angles (FR)**

(72) Inventeurs:
• **FRANC, Janyce
84000 AVIGNON (FR)**
• **PUISNEL, Christophe
84000 AVIGNON (FR)**

(74) Mandataire: **Rhein, Alain
Cabinet BREV & SUD
55 Avenue Clément Ader
34170 Castelnau-le-Lez (FR)**

(54) **SYSTEME ET PROCEDE DE DESINFECTION PAR IRRADIATION OPTIQUE D'UN PRODUIT PULVERULENT**

(57)    L'invention concerne un système de décontamination (1) d'un produit pulvérulent comportant:
- un distributeur (3) relié à une source de produit pulvérulent configuré pour distribuer le produit pulvérulent,
- au moins un transporteur (4) comportant un premier convoyeur (44) dont une première extrémité reçoit le produit pulvérulent par le distributeur (3) pour pré-étaler ledit produit en l'acheminant à une vitesse déterminée sur au moins un tapis vibrant (42) que comporte le transporteur (4) ;
- au moins un tunnel de décontamination (20) équipé d'au moins une source d'irradiation UV orientée vers le tapis vibrant (42) acheminant le produit pulvérulent au travers du tunnel de décontamination (20),
Selon l'invention le tunnel de décontamination (20) comporte des parois intérieures (23) qui sont parées au moins partiellement d'une surface réfléchissante configurée pour refléter les irradiations UV en direction du tapis vibrant (42).
    L'invention se rapporte également à un procédé décontamination.

[Fig. 1]

EP 3 884 966 A2

**Description**

[La présente invention se rapporte au domaine de la décontamination par irradiation UV. Plus particulièrement, la présente invention concerne la décontamination de produit pulvérulent par la lumière UV, et de préférence, par la lumière UV pulsée haute fréquence.

La lumière pulsée est une méthode de décontamination physique qui présente l'avantage de décontaminer efficacement des produits sans les placer au contact d'agents chimiques. A l'heure du développement de produit estampillé « biologique », l'utilisation de cette technologie attisent l'intérêts des industriels dans les secteurs agroalimentaire, pharmaceutique, cosmétique, médical etc.

Il convient de rappeler que la technologie de décontamination par lumière pulsée consiste à irradier des produits, des liquides, des environnements ou des objets à décontaminer par émission de lumière pulsée enrichie en rayonnement UV.

Le document US 4,464,336 décrit les principes généraux de la technologie d'irradiation à lumière pulsée haute fréquence. Cette technologie utilise une lampe comprenant un gaz rare, de préférence le xénon, auquel on applique une tension élevée pendant une fenêtre temporelle courte qui s'exprime en microsecondes ou en millisecondes. La tension élevée qui est appliquée aux bornes de la lampe entraine l'ionisation du gaz contenu dans la lampe, il résulte de cette ionisation, l'émission d'un flash d'intensité lumineuse élevée. Le flash lumineux correspond à un rayonnement ou une impulsion électromagnétique de lumière blanche qui présente un spectre électromagnétique allant de 180 nm à 1100 nm.

Il est aujourd'hui courant d'utiliser la technologie de la lumière pulsée pour décontaminer des emballages destinés à contenir des produits de consommation tels que des produits agroalimentaires, pharmaceutiques, cosmétiques etc. En revanche, il est moins fréquent d'utiliser cette technologie pour décontaminer les produits eux même. Bien que l'utilisation de la lumière pulsée pour décontaminer une surface ait fait ses preuves et présente d'excellents résultats, l'utilisation de cette technologie pour la décontamination des produits de consommation se heurte à des problématiques d'efficacité. Une première problématique relève de la nature de cette technologie, en effet, par définition, la décontamination optique se limite à un traitement de surface. Or, dans le cadre de préparation liquide, d'émulsion, de crème, de gels ou encore de poudre, il n'est pas possible d'exposer la totalité de la préparation aux rayonnements optiques sans étaler cette préparation selon une distribution monocouche.

De fait, l'étalement monocouche de la préparation à décontaminer constitue le premier verrou technologique auquel les industriels seront confrontés.

En ce qui concerne, les produits pulvérulents, le document FR 2 909 882 décrit un système de décontamination par lumière pulsée d'un produit pulvérulent placé en chute libre depuis une trémie faisant office de distributeur vers un réceptacle. Ce document propose de positionner une lampe UV flash entre le distributeur et le réceptacle afin de flasher le produit pulvérulent lors de sa chute libre. D'une part, ce système ne permet pas de garantir une décontamination reproductible et optimale du produit pulvérulent. En effet, il n'est pas possible de garantir que chaque particule du produit a été exposé au rayonnement UV lors de sa chute libre. D'autre part, ce système n'est pas adapté à fournir une cadence de décontamination suffisante pour satisfaire aux cadences de production industrielle qui sont supérieures à 200 kg par heure et peuvent atteindre ou dépasser la tonne par heure dans certains cas.

Une autre solution technique est proposée par le document FR 2 941 848, le système de décontamination que propose ce document comporte une trémie faisant office de distributeur qui décharge le produit pulvérulent sur un tapis vibrant. Le tapis vibrant a pour fonction de répartir la poudre selon une distribution monocouche. Le tapis vibrant est suivi d'un plan incliné fixe qui conduit la poudre sous l'effet de son inertie. Le plan incliné est en inox et est disposé traversant d'au moins une chambre de décontamination à la lumière puisée. Cependant, ce système assure une répartition monocouche pour un apport en produit pulvérulent limité. Lorsque cette limite est dépassée, il n'est plus possible d'obtenir cette répartition monocouche. Dans ces conditions, il est difficile de fournir une cadence de décontamination industrielle. De même, le plan incliné qui suit ne permet pas non plus de fournir une cadence suffisante pour satisfaire aux cadences de production industrielle. En effet, selon ce document, le produit pulvérulent parcourt un premier tapis vibrant jusqu'à un plan incliné avec pour moyen d'entrainement sa seule inertie générée par la gravité terrestre. De plus, le fait d'exposer le plan incliné en inox à de la lumière pulsée pendant de longue période entraine nécessairement une montée en température du plan incliné qu'il faudrait refroidir. Ce document ne soulève pas cette problématique et ne propose pas non plus de solution.

Par ailleurs, le plan incliné pose une problématique d'écoulement du produit pulvérulent en fonction notamment de la forme des particules qui le composent mais aussi de leur densité. Ces facteurs influencent le coefficient de frottement entre le produit pulvérulent et le plan incliné. Il est ainsi difficile de maitriser la cadence de décontamination. De plus d'autres facteurs comme la pégosité du produit peuvent influencer sur le coefficient de frottement entre le produit et le plan incliné. Par ailleurs, la construction d'un système de décontamination selon ce principe implique d'utiliser un plan incliné de plusieurs mètres de long pour obtenir une bonne répartition du produit pulvérulent. Ceci constitue un désavantage supplémentaire dans le cadre d'une installation industrielle.

Il est encore connu par le document JP H10.215836 un appareil et un procédé de stérilisation de produits pulvérulents comportant une trémie d'où s'écoule le produit pulvérulent à traiter sur un premier convoyeur vibrant d'alimentation. En aval, celui-ci déverse le produit sur un second convoyeur vibrant traversant l'unité d'irradiation UV. Les lampes UV sont disposées à l'intérieur d'un carter destiné à bloquer les rayonnements UV par rapport à l'environnement. Ce carter est monté pivotant au-dessus du convoyeur vibrant traversant l'unité d'irradiation UV. Dans sa partie inférieure, le carter comporte une vitre à quartz favorisant, au contraire, la transmission des rayonnements UV en direction de ce convoyeur vibrant sur lequel se déplace le produit pulvérulent à stériliser. Pour favoriser ce déplacement ce convoyeur est imprégné de téflon.

Au regard de cette analyse, l'état de l'art, décrit précédemment, ne fournit pas de solution afin d'intégrer une technologie de décontamination optique au sein d'une chaine de production industrielle d'un produit pulvérulent.

Dans ce contexte, la demanderesse a développé une solution technique de décontamination par irradiation optique, qui permet de décontaminer un produit pulvérulent de manière efficace tout en répondant aux exigences d'une cadence industrielle d'au moins 200 kg/h.

Dans cette optique, la présente invention se rapporte à un système de décontamination d'un produit pulvérulent comportant :

-     un distributeur relié à une source de produit pulvérulent configuré pour distribuer le produit pulvérulent,

-     un premier convoyeur alimenté en produit pulvérulent par le distributeur pour pré-étaler ledit produit en l'acheminant à une vitesse déterminée sur au moins un transporteur comportant au moins un tapis vibrant qui s'étend selon un plan horizontal et comprenant des moyens vibratoires et

-     au moins un tunnel de décontamination équipé d'au moins une source d'irradiation UV orientée vers le transporteur acheminant le produit pulvérulent en direction du tunnel de décontamination, le transporteur étant traversant du tunnel de décontamination.

Selon une première caractéristique de l'invention, le tunnel de décontamination peut comporter des parois intérieures qui sont parées au moins partiellement d'une surface réfléchissante configurée pour refléter les irradiations UV en direction du tapis vibrant. Cette caractéristique permet d'augmenter l'exposition du produit pulvérulent aux irradiations UV produites par le système d'irradiation.

Selon une deuxième caractéristique du système de décontamination, dans sa partie s'étendant dans le tunnel d'irradiation au droit d'au moins une source d'irradiation, le tapis vibrant comporte au moins une portion transparente constituée d'une plaque transparente à lumière pulsée selon une plage de longueurs d'onde comprise entre 160 et 1200 nm, le tunnel de décontamination comportant au moins une source d'irradiation UV située d'un côté par rapport à ladite plaque transparente et des moyens d'irradiation située du côté opposé par rapport à cette plaque transparente.

Selon l'invention, ces moyens d'irradiation peuvent être constitués de moyens de réflexion de rayonnement UV émis par la ou les sources d'irradiation UV disposés du côté opposé à la plaque transparente et/ou au moins une source d'irradiation UV.

Cette configuration préférentielle du système d'irradiation permet d'irradier le produit pulvérulent selon deux directions opposées lorsqu'il transite dans le tunnel de décontamination. L'exposition aux irradiations se trouve optimisée et permet d'obtenir de bons résultats de décontamination.

Comme exposé dans la description détaillée, le système selon l'invention est de fournir des cadences de décontamination de produit pulvérulent qui peuvent dépasser une capacité de traitement de l'ordre de la tonne/heure.

Selon une troisième caractéristique du système de décontamination, le distributeur comprend une trappe configurée pour distribuer le produit pulvérulent, le degré d'ouverture de la trappe étant réglable.

Selon une quatrième caractéristique, le système de décontamination comporte au moins une lampe flash constituant une source d'irradiation UV de type lumière puisée. En particulier, la lampe flash s'étend sur une distance au moins égale à la largeur du tapis vibrant.

L'invention concerne également un procédé de décontamination d'un produit pulvérulent, caractérisé en ce qu'il comporte les étapes suivantes :

-     fourniture d'un système de décontamination conforme à l'invention,
-     fourniture d'un produit pulvérulent,
-     pré-étalage du produit pulvérulent, le pré-étalage est effectué par un acheminement horizontal du produit pulvérulent sur un convoyeur qui transporte le produit pulvérulent à une vitesse déterminée en direction d'un tunnel de décontamination,
-     étalage du produit pulvérulent selon une distribution monocouche, l'étalage du produit pulvérulent est opéré au moins en partie par un mouvement vibratoire du tapis vibrant, et

- décontamination par irradiation UV du produit pulvérulent traversant le tunnel de décontamination par irradiations UV selon deux directions opposées.

Le procédé de décontamination selon l'invention contribue également à obtenir une répartition monocouche du produit pulvérulent tout en fournissant des cadences de décontamination qui peuvent varier entre 200 kg/heure et 1 tonne/heure. Il est ainsi possible d'adapter le procédé de décontamination à des cadences industrielles.

En vue d'ajuster la cadence de décontamination, le procédé de décontamination comprend une étape de calibrage d'au moins un paramètre d'acheminement et/ou d'étalement en fonction de la nature du produit pulvérulent en lui transmettant une énergie cinétique déterminée. Ainsi, le produit pulvérulent traverse le tapis vibrant et le tunnel de décontamination sous l'effet de l'énergie cinétique qu'il a emmagasinée.

De manière plus spécifique, l'étape de décontamination peut être opérée au moyen de deux sources d'irradiation UV distinctes disposées de manière opposée selon un même axe.

Avantageusement, cette décontamination intervient par une irradiation UV de lumière pulsée issue d'au moins deux lampes flashs. Selon une première possibilité, les deux lampes flashs peuvent fonctionner de manière synchronisée.

Toutefois, le procédé selon l'invention, permet également de faire fonctionner les deux lampes flashs selon un décalage temporel déterminé.

D'autres particularités et avantages apparaitront dans la description détaillée qui suit, d'un exemple de réalisation, non limitatif, de l'invention illustré par les figures 1 à 3 placées en annexe et dans lesquelles :

[Fig. 1] est une représentation latérale d'un système de décontamination d'un produit pulvérulent conforme à l'invention.

[Fig. 2] est une représentation en perspective du système décontamination de la figure 1.

[Fig. 3] est une représentation schématique du tunnel de décontamination du système de décontamination de la figure 1

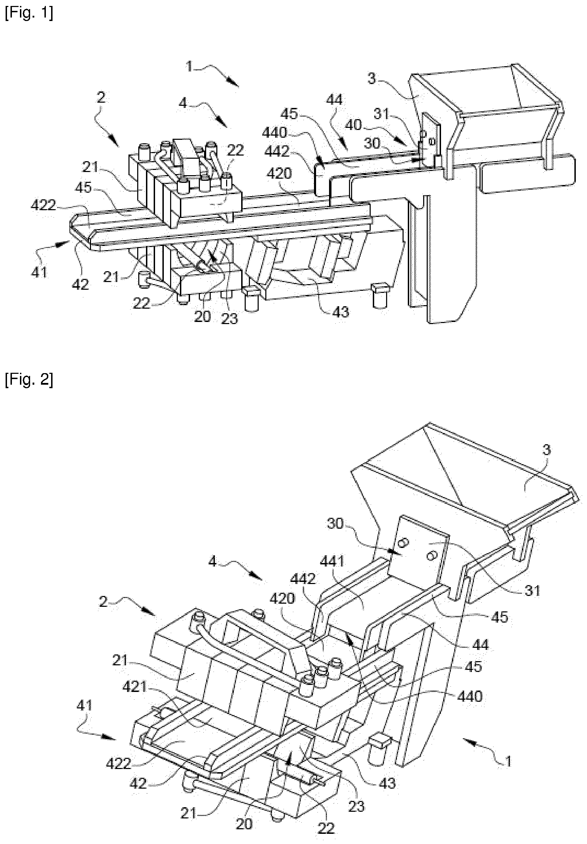

Comme illustré aux figures 1 à 3, la présente invention concerne un système de décontamination 1 d'un produit pulvérulent. Le système de décontamination est adapté à décontaminer un produit pulvérulent agroalimentaire, pharmaceutique, cosmétique etc. A titre indicatif, dans l'agroalimentaire, il est possible de décontaminer, des céréales, des farines etc. Dans le domaine de la cosmétique, l'invention permet de décontaminer les poudres qui peuvent être utilisées pour le maquillage etc.

Selon la nature des produits à décontaminer, le système de décontamination 1 comprend une source d'irradiation UV. En particulier, selon l'invention, le système de décontamination 1 peut intégrer un système d'irradiation UV continue ou un système d'irradiation 2 par lumière puisée. Il convient de noter que le choix de la source d'irradiation modifie uniquement la source d'irradiation, le reste du système de décontamination 1 ne nécessite pas de modifications structurelles particulières.

Les figures 1 à 3 illustrent plus particulièrement un exemple d'intégration d'un système de décontamination par irradiation à la lumière puisée. Le choix de la source d'irradiation dépend notamment de la nature du produit pulvérulent, mais aussi des cadences de décontamination, du germe problématique ou encore du taux de décontamination que l'on souhaite obtenir.

Par exemple, une poudre alimentaire de couleur blanche et le même produit en format concassé étaient fortement contaminée par la bactérie *Enterrococcus faecium.* Si l'objectif de décontamination est de 2 log soit une division par 100 le nombre de microorganismes *Enterrococcus faecium,* il convient de sélectionner une puissance de flash approprié pour chaque forme de produit.

[Tableau 1]

| Produit alimentaire de couleur blanche | Objectif de décontamination (en log) | Puissance du flash de lumière pulsée en J/cm$^2$ | Décontamination observée (en log) |
|---|---|---|---|
| Poudre | 2 | 2,5 | 1,4 |
| Forme concassée | 2 | 2,5 | >2 |

En effet, dans l'exemple du tableau 1 ci-dessus, pour la poudre alimentaire, 1.4 log est atteint en un flash de 2.5 J/cm$^2$ tandis que pour le produit concassé, la décontamination est supérieure à 2 log avec le même traitement. Dans cet exemple, bien que le produit soit de même nature, la densité et la forme sont différentes, entraînant des résultats de décontamination différents.

Les résultats du tableau 1, nous permettent de déduire qu'un traitement plus intense sera nécessaire pour la poudre fine que pour le produit concassé. Il est donc nécessaire d'ajuster le traitement en fonction de la nature du produit à décontaminer, de sa forme, de sa densité etc.

A cet effet, la présente invention propose un procédé et un système de décontamination 1 capables notamment d'ajuster la puissance de décontamination par lumière pulsée en fonction du produit à décontaminer.

Le système de décontamination 1 comporte au moins un tunnel de décontamination 20 dans lequel est intégré un système d'irradiation 2 à la lumière puisée. Le système d'irradiation 2 comprend au moins un module optique 21 et une lampe flash 22 de lumière pulsée tels que décrits dans la demande de brevet français déposée sous le numéro FR 19 00396. Le module optique 21 et la lampe flash 22 sont conçus afin de limiter les risques d'arc électrique entre les électrodes de la lampe flash 22. De plus, la lampe flash 22 comprend un système de refroidissement intégré qui communique avec le système de refroidissement du module optique 21. Les systèmes de refroidissement de la lampe flash 22 et du module optique 21 sont entièrement étanches à l'air et à l'eau. Ainsi, le module optique 21 est particulièrement adapté à fournir des cadences de décontamination élevées. Le module optique 21 est ainsi conçu pour produire des flashs de lumière pulsée selon une fréquence comprise entre 0,1 et 6 Hz. La fréquence de flash peut être ajustée en fonction de la vitesse à laquelle le produit pulvérulent transite dans le tunnel de décontamination 20.

Le système de décontamination 1 selon l'invention est également capable notamment de gérer la vitesse d'acheminement du produit à décontaminer vers le tunnel de décontamination 20 en fonction de la nature du produit mais aussi de la fréquence de flash de la ou des lampe(s) flash 22.

A cet effet, comme illustré aux figures 1 et 2, le système de décontamination 1 comprend un distributeur 3 configuré pour distribuer un produit pulvérulent. Ce distributeur 3 est relié, d'une part, à une source de produit pulvérulent. D'autre part, le distributeur 3 comprend une ouverture de sortie 30, au travers de laquelle, le produit pulvérulent est déversé sur au moins un transporteur qui est configuré pour conduire le produit pulvérulent vers le système d'irradiation 2.

Avantageusement, le distributeur 3 comprend une trappe 31 qui est configurée pour obstruer l'ouverture de sortie 30. Le degré d'ouverture de la trappe 31 est réglable. De fait, il est possible d'ajuster la quantité de produit pulvérulent distribuée au transporteur. Le degré d'ouverture de la trappe 31 est réglable en fonction de la cadence que l'on souhaite appliquer au système de décontamination 1. Par degré d'ouverture, l'homme du métier comprendra l'amplitude d'ouverture de la trappe 31.

Dans cet exemple, le distributeur 31 est une trémie. La trappe 31 obstrue l'ouverture de sortie 30 de la trémie. Afin d'être réglable, la trappe 31 peut être montée sur un système de glissière traditionnelle avec des moyens de verrouillage classiques. Le réglage de l'ouverture de la trappe 31 et son verrouillage en position ouverte peuvent être effectués manuellement ou à l'aide de moyens moteurs. Les moyens moteurs peuvent être pilotés par une unité numérique comprenant une mémoire et un processeur configurés pour stocker et exécuter un algorithme de paramétrage du système de décontamination 1.

Selon l'invention, le système de décontamination 1 comprend au moins un transporteur 4. Le transporteur 4 s'étend entre une première extrémité 40 et une seconde extrémité 41. La première extrémité 40 du transporteur 4 est disposée au niveau de l'ouverture de sortie 30 du distributeur 3. Selon cette configuration, la première extrémité 40 du transporteur 4 reçoit le produit pulvérulent déversé par le distributeur 3. La seconde extrémité 41 du transporteur 4 est libre dans l'exemple des figures 1 et 2. Cependant, la seconde extrémité 41 peut être suivie d'une chaine de conditionnement du produit pulvérulent décontaminé.

Comme illustré aux figures 1 à 3, le système de décontamination 1 comprend au moins un tunnel de décontamination 20 équipé d'au moins une source d'irradiation UV. Selon cet exemple, le transporteur 4 traverse le tunnel de décontamination 20. La source d'irradiation UV est orientée vers le transporteur 4 qui achemine le produit pulvérulent en direction du tunnel de décontamination 20.

En outre, le transporteur 4 comporte au moins un tronçon vibrant. Le tronçon vibrant du transporteur 4 assure l'étalement du produit pulvérulent au travers de son mouvement vibratoire de sorte à obtenir une répartition monocouche du produit pulvérulent. Comme nous l'avons évoqué dans la présentation du problème technique, il est primordial que le produit pulvérulent soit réparti selon une monocouche afin d'optimiser l'exposition des particules de produit pulvérulent au rayonnement irradiant lorsque ces particules traversent le tunnel de décontamination 20.

Selon l'invention, le transporteur 4 comporte au moins un tapis vibrant 42 qui s'étend selon un plan H. Ici, le plan H est un plan horizontal. On entend ici par plan horizontal, un plan perpendiculaire au vecteur de gravité G.

Par ailleurs, le tapis vibrant 42 vibre selon une fréquence et une amplitude déterminée. La fréquence et l'amplitude de vibrations du tapis vibrant 42 peuvent être paramétrées en fonction de la cadence de décontamination et du type de produit pulvérulent que l'on souhaite décontaminer.

Dans cet objectif, le tapis vibrant 42 est équipé de moyens vibratoires 43. Les moyens vibratoires 43 peuvent consister en un vibreur industriel de type électrique, pneumatique, électromagnétique etc. Les moyens vibratoires 43 peuvent être paramétrés selon deux paramètres, l'amplitude de vibration et la fréquence de vibration. A titre indicatif, la fréquence de vibration peut être paramétrée entre 30 Hz et 130 Hz. Dans le même temps, l'amplitude de vibration peut être paramétrée selon un nombre déterminé de paliers.

De fait, le tapis vibrant 42 assure une double fonction. D'une part, le tapis vibrant 42 conduit le produit pulvérulent au travers du tunnel de décontamination 20. D'autre part, le tapis vibrant 42 est configuré pour étaler le produit pulvérulent au travers de son mouvement vibratoire. Ceci assure une répartition monocouche du produit pulvérulent.

De préférence selon l'invention, le transporteur 4 comporte un premier convoyeur 44 alimenté en produit pulvérulent par le distributeur 3 pour pré-étaler ledit produit en l'acheminant à une vitesse déterminée sur le tapis vibrant 42. Le premier convoyeur 44 est donc intercalé entre le distributeur 3 et le tapis vibrant 42 et s'étend entre une première extrémité et une seconde extrémité 46. La première extrémité du premier convoyeur 44 correspond à la première extrémité 40 du transporteur 4. Selon cette configuration, la première extrémité du convoyeur 44 reçoit le produit pulvérulent délivré par le distributeur 3. A l'opposé, la seconde extrémité 440 du convoyeur 44 est disposée de façon adjacente à une extrémité de réception du tapis vibrant 42 (illustré aux figures 1 et 2).

Le premier convoyeur 44 s'étend également selon un plan horizontal. Dans cet exemple, le convoyeur 44 s'étend selon un plan horizontal surélevé par rapport au plan horizontal du tapis vibrant 42 (illustré à la figure 1).

Le premier convoyeur 44 comporte un tapis roulant 441 tournant à une vitesse déterminée. La vitesse du tapis roulant 441 du convoyeur 44 peut être comprise entre 1 à 40 m/min. La vitesse du convoyeur 44 est paramétrée en fonction de plusieurs facteurs, tels que la cadence de décontamination, la nature et/ou la forme du produit pulvérulent, la quantité de produit délivré par le distributeur etc.

Le premier convoyeur 44 assure deux fonctions. D'une part, le convoyeur 44 achemine le produit pulvérulent à une vitesse déterminée vers le tapis vibrant 42. D'autre part, le convoyeur 44 pré-étale le produit pulvérulent déversé par le distributeur 3.

Comme illustré aux figures 1 et 2, le tapis vibrant 42 et le premier convoyeur 44 s'étendent longitudinalement entre leurs extrémités respectives. En outre, le transporteur 4 comporte deux rebords latéraux 45. Le tapis vibrant 42 et le premier convoyeur 44 s'étendent respectivement de façon transversale entre les bords latéraux 45. En particulier, les rebords latéraux 45 sont saillants du tapis vibrant 42 et du tapis roulant 441 du premier convoyeur 44. Ceci en vue de prévenir des projections de produit pulvérulent à l'extérieur du transporteur 4. Il est à noter que les rebords latéraux 45 du premier convoyeur 44 comportent respectivement une extension 442. Cette extension 442 s'étend au-delà de la seconde extrémité 440 du premier convoyeur 44. Ces extensions 442 sont utiles pour prévenir la projection de produit pulvérulent hors du transporteur 4, dès lors que le premier convoyeur 44 s'étend sur un plan surélevé par rapport au plan dans lequel s'étend le tapis vibrant 42.

Selon l'invention, la distance qui sépare deux bords latéraux 45 d'un même convoyeur 42, 44 définit la largeur du convoyeur 42, 44. Dans cet exemple, la largeur du tapis vibrant 42 est supérieure à la largeur du premier convoyeur 44. A titre d'exemple, la largeur du tapis vibrant 42 peut être comprise entre 150 mm et 400 mm. De son côté, la largeur du premier convoyeur 44 peut être comprise entre 75 mm et 200 mm. Dans tous les cas, la largeur du convoyeur de vibrant 42 est supérieure à la largeur du premier convoyeur 44. En effet, il est préférable que la largeur du tapis vibrant 42 soit au moins 1,3 fois supérieure à la largeur du premier convoyeur 44. De préférence, la largeur du tapis vibrant 42 est au moins 1,5 fois supérieure à la largeur du premier convoyeur 44.

Le fait que le tapis vibrant 42 soit plus large que le premier convoyeur 44 participe à optimiser l'étalement du produit pulvérulent selon une répartition monocouche avant que le produit pulvérulent ne traverse le tunnel de décontamination 20. Selon l'invention, le tunnel de décontamination 20 comprend au moins une source d'irradiation UV. Dans le présent exemple, la source de radiation UV est une lampe flash 22 de lumière puisée. Dans cet exemple, la lampe flash 22 s'étend sur une distance au moins égale à la largeur du tapis vibrant 42. Cette caractéristique permet d'optimiser la surface d'exposition aux rayonnements de la lampe flash 22.

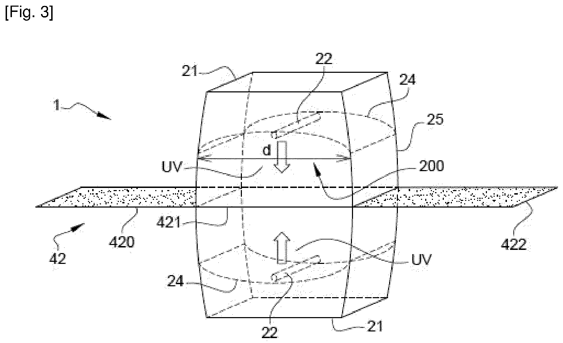

Afin d'optimiser la puissance d'un flash de la lampe flash 22, le tunnel de décontamination 20 comporte des parois intérieures 23 qui sont parées, au moins partiellement, d'une surface réfléchissante configurée pour refléter les irradiations UV en direction du tapis vibrant 42. En pratique, les parois intérieures 23 sont entièrement parées d'une surface réfléchissante afin d'optimiser la puissance disponible d'un flash de la lampe flash 22. Les parois intérieures 23 réfléchissantes constituent un réflecteur 24 (illustré à la figure 3). Comme évoqué au préalable, la demande de brevet français déposée sous le numéro FR 19 00396 décrit le module optique 21 et la lampe flash 22 qui sont utilisés pour former le tunnel de décontamination 20.

Dans l'exemple illustré à la figure 2, le tapis vibrant 42 comporte au moins une portion transparente 421 disposée entre des portions opaque 420, 422. Cette portion transparente 421 est constituée d'une plaque transparente à lumière pulsée selon une plage de longueurs d'onde comprise entre 160 et 1200 nm..

Le tunnel de décontamination 20 comporte au moins une source d'irradiation UV située supérieurement par rapport à ladite plaque transparente 421. et des moyens d'irradiation située inférieurement à cette dernière.

Selon une configuration préférée, la plaque transparente 421 du trapis vibrant 42 s'étend sensibnlement sur toute la longueur du tunnel de décontamination 20 s'étend de part et d'autre de la portion transparente 421. Le tunnel de décontamination 20 comporte au moins une source d'irradiation UV située supérieurement ou inférieurement par rapport à la plaque transparente.

De préférence, le tunnel de décontamination 20 comporte au moins une source d'irradiation UV située supérieurement par rapport à ladite plaque transparente 421 et des moyens d'irradiation situés inférieurement à cette dernière ou inversement.

En particulier, les moyens d'irradiation disposés d'un côté de la plaque transparente 421 peuvent être définis par des moyens de réflexion de rayonnement UV émis par la source d'irradiation UV disposée du côté opposé à cette plaque transparente 421.

Avantageusement, le tunnel de décontamination 20 comporte deux sources d'irradiation disposées respectivement supérieurement et inférieurement par rapport à la portion transparente 421. Selon un mode de réalisation préférentiel de l'invention illustré à la figure 3, le tunnel de décontamination 20 comprend deux modules optiques 21 qui sont disposés de manière symétrique par rapport à la portion transparente 421. De cette façon, il est possible d'exposer le produit pulvérulent à des irradiations UV provenant des parties supérieures et inférieures du tunnel de décontamination. Selon cette configuration, chaque module optique 21 est équipé d'une lampe flash 22 et d'un réflecteur 24. Chaque module optique 21 est disposé de manière à orienter les irradiations UV en direction de la portion transparente 421 du tapis vibrant 42. A cet effet, chaque module optique 21 comporte une ouverture longitudinale 200 qui s'étend selon un axe parallèle à l'axe de lampe flash 22. Cette ouverture 200 contribue à orienter les irradiations en fonction d'au moins une partie de la portion transparente 421. En fonction de la nature du produit pulvérulent à décontaminer, il est possible de faire varier l'amplitude de l'ouverture 200. L'amplitude de l'ouverture 200 est ajustable selon un axe d transversal de l'axe longitudinal de la lampe flash 22 (illustré à la figure 3). De préférence, l'amplitude de l'ouverture 200 est ajustable entre 15% et 100% de la distance d. Dans cet exemple, la distance d correspond à la largueur interne maximale du module optique 21.

A titre indicatif, pour un module optique 21 qui possède une largeur interne maximale de 120 mm, l'amplitude de l'ouverture 200 peut être ajustée entre 18 mm et 120 mm. Ici, 120 mm correspondant à 100% de l'amplitude possible alors que 18 mm correspond à 15 % de l'amplitude d'ouverture 200 possible.

La variation de l'amplitude d'ouverture 200 permet d'augmenter ou de réduire l'exposition de la portion transparente 421 aux irradiations UV. Cela constitue un paramètre de réglage et/ou de conception du système selon l'invention pour optimiser la décontamination en fonction d'une cadence industrielle ou inversement.

Les deux lampes flash 22 peuvent fonctionner simultanément ou selon un décalage temporel déterminé. Le fonctionnement simultané ou en décalé des lampes flashs 22 peut-être paramétré en fonction des cadences que l'on souhaite obtenir et de la nature du produit pulvérulent.

Il est à noter qu'un fonctionnement simultané des deux lampes flashs 22 permet d'augmenter l'énergie reçue par le produit pulvérulent en seul flash. La lumière pulsée n'est pas additive. Il est souvent préférable d'avoir un flash ultra intense plutôt que deux flashs moins intenses.

Néanmoins, quand un seul flash permet d'obtenir les niveaux de décontamination souhaités, un fonctionnement en décalé peut-être envisagé afin de doubler les cadences de production. Par ailleurs, même si les lampes flash 22 fonctionnent selon un décalage temporel, le réflecteur d'un premier module optique 21 reflète le flash produit par la lampe flash 22 du second module optique 21. Cette configuration avantageuse permet d'optimiser les niveaux de décontamination du produit pulvérulent.

Selon l'invention, le tunnel de décontamination 20 est disposé dans une chambre noire 25 (illustré à la figure 3). La chambre noire permet de confiner les rayonnements UV au sein du tunnel de décontamination 20. Ceci permet de préserver l'intégrité physique des opérateurs en charge de la chaine de production.

De manière générale, l'invention permet d'optimiser la surface d'exposition du produit pulvérulent au travers de sa répartition monocouche sur le transporteur 4. Par ailleurs, l'usage d'un transporteur 4 horizontal à vitesse réglable permet de maitriser précisément les cadences de décontamination d'un produit pulvérulent en fonction de sa nature, et de fait, en fonction de son coefficient de frottement avec le tapis roulant 441 et le tapis vibrant 42.

Le tableau 2 ci-dessous illustre la capacité du système de décontamination 1 à maitriser les cadences de décontamination. Dans le premier exemple de ce tableau, une cadence de 800 kg de riz par heure est obtenue pour un riz qui présente une densité de 833 g/l. Pour obtenir une telle cadence relative à la densimétrie de ce produit, l'exemple 1 utilise une fréquence de flash de 4,44 Hz alors que le tapis roulant 441 tourne à une vitesse de 24 m/min.

Le tableau 2 illustré un deuxième exemple la décontamination d'une gomme arabique selon une cadence de 200 kg/h. La gomme arabique possède une densité de 1425 g/l. De fait, le système de décontamination est adapté à ces paramètres, on notera notamment une réduction du degré d'ouverture du distributeur 3, une réduction de la largeur du tapis vibrant 42, une réduction de la vitesse du tapis roulant 441, une réduction de la fréquence de flash. La surface de décontamination correspond au produit de l'amplitude de l'ouverture 200 du module optique 21 avec la largeur du tapis vibrant 42. Lorsque l'on a un produit plus dense, la surface décontamination est réduite afin de concentrer les irradiations UV. Par ailleurs, la réduction de la vitesse du tapis roulant 441 permet d'augmenter le temps de passage du produit dans le tunnel de décontamination 20.

*[Tableau 2]*

| Paramètres | Exemple 1- décontamination du riz | Exemple 2 - Décontamination Gomme arabique |
|---|---|---|
| Quantité produit (en kg) par heure | 800 | 200 |
| Densité produit en g/l | 833 | 1425 |
| Ouverture du distributeur en mm | 400 | 200 |
| Largeur tapis vibrant en mm | 400 | 200 |
| Surface de décontamination en dm$^2$ | 3,6 | 1.8 |
| Amplitude d'ouverture du module optique en mm | 90 | 90 |
| Fréquence des flashs en Hz | 4,44 | 1.85 |
| Vitesse tapis roulant en m/min | 24 | 10 |
| Temps de transite du produit sur un convoyeur d'un mètre de long en seconde | 2,5 | 6 |

De plus, l'invention permet également maitriser la compacité du système de décontamination 1. En effet, selon l'invention le transporteur 4 s'étend sur une distance comprise entre 1 m et 2 m. Plus précisément, le premier convoyeur 44 peut s'étendre sur une distance comprise entre 300 mm et 700 mm. De son côté, le tapis vibrant 42 peut s'étendre selon une distance comprise entre 700 mm et 1300 mm. Ces valeurs sont relativement modestes en comparaison du système de décontamination de l'art antérieur qui requiert un convoyeur de plusieurs mètres de long pour obtenir une répartition monocouche du produit pulvérulent.

La présente invention concerne également, un procédé de décontamination d'un produit pulvérulent. Ce procédé de décontamination utilise le système de décontamination 1 conforme de à l'invention pour décontaminer un produit pulvérulent.

Dans ce contexte, le procédé de décontamination comporte une étape de fourniture d'un système de décontamination 1 conforme à l'invention. Afin de démarrer le procédé de décontamination, une étape de fourniture d'un produit pulvérulent est opérée. Selon l'invention, le distributeur 3 est alors alimenté au travers d'une source de produit pulvérulent qui peut être une cuve de conservation ou alors une chaine production.

Au préalable le degré d'ouverture de la trappe 31 est ajustée en fonction de la cadence de décontamination que l'on souhaite obtenir et de la nature du produit pulvérulent. La densimétrie du produit pulvérulent est également un des facteurs qui peut être pris en compte pour le réglage du degré d'ouverture de la trappe 31.

Le procédé comprend au moins une étape d'acheminement horizontal du produit pulvérulent sur un transporteur 4. Le transporteur 4 achemine le produit pulvérulent en direction d'un tunnel de décontamination 20. L'acheminement horizontal est opéré selon au moins une vitesse déterminée.

Le procédé comprend une étape d'étalage du produit pulvérulent selon une distribution monocouche. L'étalage du produit pulvérulent est opéré au moins en partie par un mouvement vibratoire du transporteur 4.

Avantageusement, les étapes d'étalage et d'acheminement horizontal sont simultanées. En pratique, les étapes d'étalage est d'acheminement horizontal sont opérés par le tapis vibrant 42. D'une part, le mouvement vibratoire du tapis vibrant 42 étale le produit pulvérulent selon une répartition monocouche, et d'autre part, la première portion opaque 420 du tapis vibrant 42 conduit le produit pulvérulent de sorte à ce qu'il traverse le tunnel de décontamination 20.

En outre, le procédé de décontamination peut comprendre une étape de pré-étalage du produit pulvérulent. Le pré-étalage est effectué par acheminement mécanique horizontal du produit pulvérulent à une vitesse déterminée en direction du tunnel de décontamination 20. En pratique, le pré-étalage est opéré par le premier convoyeur 44. En effet, ce dernier pré-étale le produit pulvérulent déversé par le distributeur 3 au travers de la vitesse de rotation de son tapis roulant 441. Le tapis roulant 441 transmet au produit pulvérulent une énergie cinétique qui lui permet de traverser le tapis vibrant 42 et le tunnel de décontamination 20 par glissement. Lorsque le produit pulvérulent parvient à la portion transparente 421 du tapis vibrant 42, le produit pulvérulent traverse alors le tunnel de décontamination 20 où il est exposé à des irradiations UV. En sortie du tunnel de décontamination 20, le produit pulvérulent, toujours sous l'effet de son inertie, traverse la seconde portion opaque 422 du tapis vibrant qui l'achemine vers une cuve de stockage ou une chaine de conditionnement.

Le procédé comporte ainsi une étape de décontamination par irradiation UV du produit pulvérulent lorsque ce dernier traverse le tunnel de décontamination 20. Comme illustré à la figure 3, lors de l'étape de décontamination, le produit pulvérulent reçoit des irradiations UV selon deux directions opposées

Selon un mode de réalisation avantageux, le produit pulvérulent reçoit des irradiations UV selon deux directions opposées provenant de deux sources d'irradiation UV distinctes disposées de manière opposée selon un même axe.

Dans le cas d'une lampe UV continue, l'étape de décontamination est opérée par exposition aux irradiation UV d'au moins une lampe UV et des rayons reflétés par la surface réfléchissante du tunnel de décontamination 22. Cependant, il est possible de disposer une lampe UV inférieurement et supérieurement à la portion transparente 421 afin d'exposer le produit pulvérulent à deux sources de rayonnement UV provenant de deux directions opposées.

Dans le cas d'une lampe flash 22 de lumière pulsée, l'étape de décontamination est réalisée par des flashs de lumière pulsée émis selon une fréquence comprise entre 1 Hz et 6 Hz. De la même façon, chaque réflecteur 24 du tunnel de décontamination 20 est configuré pour refléter les rayonnements de lumière pulsée résiduelle en direction de la portion transparente 421 sur laquelle transite le produit pulvérulent. Les rayons UV reflétés par les réflecteurs 24 ne sont pas représentés à la figure 3. De préférence, la fréquence de flashs d'une lampe flash 22 de lumière pulsée est comprise entre 3 Hz et 5 Hz. Lorsque le tunnel de décontamination 20 comporte deux lampes flashs 22 disposées inférieurement et supérieurement à la portion transparente 421, l'étape de décontamination peut être opérée par un fonctionnement simultané des lampes flashs 22.

Comme illustré dans le tableau 3 ci-dessous, la fréquence de flash simultanée des deux lampes 22 varie en fonction de la vitesse du tapis roulant 44. Ceci constitue un paramètre de réglage important afin que le produit pulvérulent soit exposé à une quantité suffisante d'irradiation UV.

[Tableau 3]

| Paramètres du tapis vibrant | | Temps de passage dans le tunnel de décontamination | Vitesse Tapis roulant | Cadence flash |
|---|---|---|---|---|
| Fréquence en Hz | Amplitude de vibration | En seconde | En m/min | En Hz |
| 59 | 206 | 1,3 | 19,5 | 0,31 |
| 59,2 | 181 | 1,6 | 15,8 | 0,38 |
| 59,3 | 168 | 1,7 | 14,5 | 0,41 |
| 59,5 | 159 | 2,0 | 12,7 | 0,47 |
| 59,4 | 149 | 2,3 | 10,9 | 0,55 |
| 59,6 | 138 | 3,0 | 8,5 | 0,70 |

Toutefois, dans cette configuration, il est également possible de faire fonctionner les deux lampes flashs 22 de façon décalée temporellement. Le décalage temporel peut être intéressant selon la cadence de transite du produit pulvérulent pour augmenter le temps d'exposition aux flashs de lumière pulsée ou pour doubler les cadences de production.

En effet, il est possible d'augmenter le temps d'exposition de la portion transparente 421 en faisant fonctionner les deux lampes flashs 22 alternativement l'une après l'autre. Afin d'optimiser ce temps d'exposition, le décalage temporel Dt de la cadence de flash entre les deux lampes flashs 22 peut correspondre à la moitié de la période $T_{lampe}$ de chaque flash.

$$Dt = \frac{Tlampe}{2}$$

Avec Dt : le décalage temporel entre les deux lampes flashs 22, et

$T_{lampe}$ = la période de chaque flash

A titre indicatif, s'il est nécessaire d'utiliser une fréquence de flash de 3,5 Hz pour chaque lampe flash 22, la période $T_{lampe}$ de flash correspond alors à 285 ms. Dans ce contexte, selon la formule ci-dessus, le décalage temporel Dt est de 142,5 ms.

En sus, le procédé de décontamination peut comprendre une étape de calibrage d'au moins un paramètre d'acheminement et/ou d'étalement en fonction de la nature du produit pulvérulent. Le paramètre d'acheminement et/ou d'étalement peut consister dans la vitesse d'acheminement, l'intensité des mouvements vibratoires, le degré d'ouverture de la trappe 31, la vitesse de pré-étalage.

Par ailleurs, lors de l'étape de pré-étalage, le premier convoyeur 44 transmet au produit pulvérulent une énergie cinétique déterminée, le produit pulvérulent traversant le tapis vibrant 42 et le tunnel de décontamination 20 sous l'effet de l'énergie cinétique qu'il a emmagasinée.

Bien entendu, il est également possible de faire varier la puissance de flash de la lumière puisée. Les différents exemples décrits précédemment, utilisent une lampe flash 22 capable de fournir une puissance d'irradiation de 2.5 $J/cm^2$. La puissance de flash est ajusté en fonction de différentes propriété du produit pulvérulent telles que sa densimétrie, ou sa pégosité.

**Revendications**

1. Système de décontamination (1) d'un produit pulvérulent comportant :

   - un distributeur (3) relié à une source de produit pulvérulent configuré pour distribuer le produit pulvérulent,
   - au moins un transporteur (4) comportant un premier convoyeur (44) dont une première extrémité reçoit le produit pulvérulent par le distributeur (3) pour pré-étaler , ledit produit en l'acheminant à une vitesse déterminée sur au moins un tapis vibrant (42) que comporte le transporteur (4),
   - au moins un tunnel de décontamination (20) équipé d'au moins une source d'irradiation UV orientée vers le tapis vibrant (42) acheminant le produit pulvérulent au travers du tunnel de décontamination (20),

   **caractérisé en ce que** le tunnel de décontamination (20) comporte des parois intérieures (23) qui sont parées au moins partiellement d'une surface réfléchissante configurée pour refléter les irradiations UV en direction du tapis vibrant (42).

2. Système de décontamination (1) selon la revendication 1, **caractérisé en ce que** le tapis vibrant (42) comporte au moins une portion transparente (421) constituée d'une plaque transparente à lumière pulsée selon une plage de longueurs d'onde comprise entre 160 et 1200 nm, le tunnel de décontamination (20) comportant une source d'irradiation UV située d'un côté par rapport à ladite plaque transparente (421) et des moyens d'irradiation située du côté opposé par rapport à cette plaque transparente (421).

3. Système de décontamination (1) selon la revendication 2, **caractérisé en ce que** les moyens d'irradiation sont constitués de moyens de réflexion de rayonnement UV émis par la ou les sources d'irradiation UV disposés du côté opposé à la plaque transparente (421).

4. Système de décontamination (1) selon la revendication 2 ou 3, **caractérisé en ce que** le tunnel de décontamination (20) comporte deux sources d'irradiation UV disposées inférieurement et supérieurement par rapport à la portion transparente (421).

5. Système de décontamination (1) selon l'une des revendication 1 à 4, **caractérisé en ce que** le distributeur (3) comprend une trappe (31) configurée pour distribuer le produit pulvérulent, le degré d'ouverture de la trappe (31) étant réglable.

6. Système de décontamination (1) selon l'une des revendication 1 à 5, **caractérisé en ce qu'**il comporte au moins une lampe flash (22) constituant une source d'irradiation UV de type lumière puisée.

7. Système de décontamination (1) selon la revendication 6, **caractérisé en ce que** la lampe flash (22) s'étend sur une distance au moins égale à la largeur du tapis vibrant (42).

8. Procédé de décontamination d'un produit pulvérulent, **caractérisé en ce qu'**il comporte les étapes suivantes :

   - fourniture d'un système de décontamination (1) selon l'une des revendications 1 à 7,
   - fourniture d'un produit pulvérulent,
   - pré-étalage du produit pulvérulent, le pré-étalage est effectué par une acheminement mécanique horizontal du produit pulvérulent sur un premier convoyeur (44) qui transporte le produit pulvérulent à une vitesse déterminée en direction d'un tunnel de décontamination (20),
   - étalage du produit pulvérulent selon une distribution monocouche, l'étalage du produit pulvérulent est opéré par un mouvement vibratoire du tapis vibrant (42),
- et décontamination du produit pulvérulent traversant le tunnel de décontamination (20) par irradiation UV selon deux directions opposées

9.  Procédé de décontamination selon la revendication 8, **caractérisé en ce qu'**il comprend une étape de calibrage d'au moins un paramètre d'acheminement et/ou d'étalement en fonction de la nature du produit pulvérulent.

10. Procédé de décontamination selon l'une des revendications 8 et 9, **caractérisé en ce que** le premier convoyeur (44) transmet au produit pulvérulent une énergie cinétique déterminée, le produit pulvérulent traversant le tapis vibrant (42) et le tunnel de décontamination (20) sous l'effet de l'énergie cinétique qu'il a emmagasinée.

11. Procédé de décontamination selon l'une des revendications 8 à 10, **caractérisé en ce que**, lors de l'étape de décontamination, le produit pulvérulent reçoit des irradiations UV selon deux directions opposées provenant de deux sources d'irradiation UV distinctes disposées de manière opposée selon un même axe.

12. Procédé de décontamination selon la revendication 11, **caractérisé en ce que** l'étape de décontamination est opérée par une irradiation UV de lumière pulsée issue d'au moins deux lampes flashs (22).

13. Procédé de décontamination selon la revendication 12, **caractérisé en ce que** les deux lampes flashs (22) fonctionnent de manière synchronisée.

14. Procédé de décontamination selon la revendication 12, **caractérisé en ce que** les deux lampes flashs (22) fonctionnent selon décalage temporel déterminé.

EP 3 884 966 A2

[Fig. 1]

[Fig. 2]

[Fig. 3]

**EP 3 884 966 A2**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- US 4464336 A
- FR 2909882
- FR 2941848
- JP H10215836 B
- FR 1900396